# EUROPEAN PATENT SPECIFICATION

(11) **EP 4 580 699 B1**
(45) Date of publication and mention of the grant of the patent: **23.09.2026**
(21) Application number: 23764326.7
(22) Date of filing: 31.08.2023
(51) Int. Cl.: A61M 5/14, A45F 3/04, A61J 1/10, A61J 1/14, A61J 7/00, A61J 15/00, A45C 11/00, A45C 13/02

(54) **PUMP SUPPORT FOR PORTABLE PUMP**
PUMPENTRÄGER FÜR TRAGBARE PUMPE
SUPPORT DE POMPE POUR POMPE PORTABLE

(30) Priority: 02.09.2022 WO PCT/EP2022/074437
(43) Date of publication of application: 09.07.2025
(73) Proprietor: N.V. Nutricia, 2712 HM Zoetermeer (NL)
(72) Inventor: VAN KINDEREN, Sencha, 3584 CT Utrecht (NL)
(74) Representative: Nederlandsch Octrooibureau
(86) International application number: PCT/EP2023/073965
(87) International publication number: WO 2024/047198

(56) References cited:
- EP-B1- 3 181 112
- EP-B1- 3 720 521
- US-A- 5 879 143

## Description

### Field of the invention

The present invention relates to a support for a portable pump and associated container for transport and administration of enteral food or other liquids.

### Background art

Patients with certain medical problems may require having enteral food or liquids administered in predetermined doses throughout the day. Containers holding the enteral food or liquid come in many different structures and shapes, having different sized and shaped necks and screw fittings. Stands may be used to allow for a container, such as a bottle or pouch, to be hung such that the enteral food or liquid can be fed through a feeding set under gravity or by pump. The feeding sets are available in at least the following, different configurations:
- with drip chamber - for use with gravity
- with drip chamber - for use with a pump
- without drip chamber - for use with a pump

In this context, feeding set is understood to generally denote an assembly of tubing with the requisite connectors for connecting to the container on the one side and to the patient feeding tube at the other side, with or without drip chamber. Furthermore, it must be noted that drip chambers may also come in a range of sizes. A clamp apparatus configured to couple a medical device to a support pole is disclosed in EP 3181112B1. An adaptor designed to secure an infusion-type pump to an enclosure for delivery of fluids is disclosed in US 5879143A1,

In most cases, patients with no or limited mobility will use a feeding set with a drip chamber. The drip chamber is situated between the container and the tubing, acting as a micro-bacterial barrier, preventing bacteria traveling up into the container where they can multiply and become harmful to very weak patients. An additional advantage of using a drip chamber is that the drip chamber offers visual feedback of enteral food or liquid being fed through the tubing.

The conventional stands referred to above are generally in the form of infusion poles or may be integrated into a bed frame. This has as a drawback that they are very limiting to the freedom of movement for patients who are not bedridden. These patients may be better served with a portable carrier, which can be used as both a stationary frame as well as a mobile frame, carried in a backpack.

One assembly for enteral feeding sets that can be carried in a backpack is shown in WO2019/112429. This assembly comprises a protective shell for a container of enteral feeding solution, that fits within the backpack together with a pump. The pump can be used to deliver the feeding solution while on the move. Alternatively, the container can be elevated and hung from an adjustable container stabilizer
for delivery by gravity. This solution has been found very versatile in giving patients considerable freedom. The pump is held by insertion vertically between support ribs integrally formed with the shell and can be removed, if required, e.g for programming. It has however been noted that for each variation in pump design, a different shell must be provided. Furthermore, although the pump is well retained against horizontal movement, it is free to move vertically and can fall out from the support ribs if the backpack or carrier is inverted.

It would therefore be desirable to provide a carrier that could at least partly obviate the above inconveniences.

### Summary of the invention

The invention is defined by independent claims 1 and 13. Further advantageous embodiments of the invention are defined in the dependent claims. According to the invention, there is provided a carrier for transporting and administering enteral food or other liquids. The carrier comprises a shell, having a bottom tray, a back plate and two side plates and a feed pump holding element. Support elements are provided for holding a container in place within the shell, the support elements comprising an adjustable container stabilizer, extendable vertically with respect to the back plate. The feed pump holding element comprises a removable front plate, releasably secured between the side plates and having a front face onto which a feed pump can be attached. The shell may also have an open upper side, allowing a container to extend out of the shell, above a top edge of the back plate and/or the side plates. As a consequence of the removable front plate, a range of different pumps may be fastened to the front plate and thus secured to the shell of the carrier. The invention also relates to such a front plate, which acts as an adapter between the pump and the carrier providing for greater versatility of design. It also ensures greater security, since the pump may be retained to the front plate in a manner that prevents it from releasing if the carrier is inverted or shaken. The front plate can also be used independently as a table stand for the pump.

The front plate may be of any suitable shape that allows it to connect to the pump and to the shell. It may be square, rectangular, trapezoidal, triangular, oval or any other suitable shape. It may be flat or profiled to match the rear surface of the pump. It may be provided with protuberances and apertures e.g for location purposes that may be customised to the intended pump. Preferably a screw connector is provided on a rear surface of the front plate for releasably attaching the feed pump. A number of screw connectors may be provided or a number of locations may be provided at which a screw may be inserted in order to cater for a range of different feed pumps. It will be understood that other forms of connection may also be envisaged including bayonet, hook and eye fasteners, studs and the like.

According to the invention, the plate comprises an upper edge, a lower edge and two side edges, the side edges being provided with clips for engaging the side plates. The side plates of the shell may be provided with left and right apertures and the clips may comprise resilient fingers arranged to extend into the apertures from within the shell. One clip on each side edge may be sufficient but it is not excluded that further clips may be provided. In an embodiment, the resilient fingers may extend through the shell from the inside towards the outside. They may be accessible from an outside of the shell to release them from the apertures in order to disengage the front plate.

According to the invention, clips are provided that extend rearwardly from the front plate as feet on which the front plate can stand at an angle once removed from the shell. This allows the front plate to be used as a stand on which the pump can be stood once removed from the backpack and carrier. Depending on the position and length of the feet, the plate can stand on a surface such as a table at an angle of from 10 degrees to 45 degrees. This can considerably improve viewing comfort of the display of such pumps. The front plate may stand on its feet and on the lower edge or a corresponding lower edge of the feed pump. It will be understood that the lower edge need not be a straight edge joining the two side edges and may e.g. be bowed downwards in the middle. A central point of contact may better serve as a third foot allowing the front plate to stand as a tripod on less even surfaces.

According to the invention, the clips are located adjacent to the upper edge and the front plate is further attached to the shell adjacent to its lower edge. Providing three or more points of connection assists in stably retaining the front plate, especially when the pump is relatively heavy. According to the invention, this connection at the lower edge is a pivotable connection. In an example not according to the invention, the front plate need not be completely removable from the shell and may merely pivot to an open position e.g. for connection of the pump. Nevertheless, a completely detachable front plate is preferred as this permits its use as a stand.

Pivoting of the front plate may be achieved by any suitable means, including additional hinge elements. Most preferably, no additional elements are provided and the hinge is integrally formed directly with the front plate and the shell respectively. In one embodiment, the front plate comprises left and right trunnions adjacent its lower edge that engage in bearings formed in the side plates. The trunnions are effectively pins that are received by curved bearing surfaces, all being aligned along a common axis.

The support elements may include a height adjustable lower support element that allows for a height between the bottom tray and support element to be adjusted such that sufficient space can be made available for various sized and shaped container necks and feeding sets with or without drip chambers. There may also be a height adjustable rear support element offering further support and stability to larger containers and/or higher positioned containers. Additionally, the support elements may comprise a container retaining element that also adapts together with the other support elements or to the size of a longer container. The height adjustable support elements thus facilitate the use of various types of feeding sets, with and without drip chamber. The adjustability of the support elements allows the carrier to be adjusted to be as compact as possible for the container and feeding set it is carrying, ensuring the carrier can comfortably be carried around in a bag. The bag can be a pouch, net, backpack, frontpack, beltpack, holdall, etc.

The container retaining element further ensures the container is secured in place within the carrier, and able to withstand loads occurring from being mobile. The container retaining element may be a strap, band or web. However, other types of retaining elements such as click connections or hook and eye type fasteners can also be used..

The carrier can be used as a mobile stand inside a dedicated bag, specifically designed to fit with the carrier, or used in a bag of personal choice. The shell of the carrier hereby serves as a protective shell for the container and also for the pump and feeding set. The bottom tray may be provided with openings should this be required e.g. for passage of cables or tubes. In a preferred embodiment the bottom tray is embodied as a spillage tray, without openings. Thus the spillage tray is an integral part of the shell, shaped as an indented region, e.g. dish shaped, or with upstanding edges. Through the presence of the spillage tray, the shell may retain spilled enteral food or liquid, helping ensure that any bag used for carrying will remain clean. Furthermore, the bottom tray of the shell is designed to provide a stable base for the carrier inside a bag as well as on any flat surface. As a result, the carrier is also suitable for use as a stationary frame on for example a table or a nightstand. The lower edge of the front plate may extend forwards of the spillage tray, whereby the pump is held forwards of the spillage tray. Alternatively, the front plate may be located deeper within the shell whereby the pump is held within the shell above the spillage tray.

According to another embodiment, the lower support element comprises a container rest shelf, extending between the side plates, which is repositionable, such that a distance between the shelf and the bottom tray is variable. A lowest position of the shelf may be aligned with an upper edge of the front plate. The shelf may also extend to and/or be affixed to the back plate. The shelf provides a stable support to any container whereby loads resulting from the weight of the container and any additional loads resulting from being mobile are distributed from the container's contact surface to the shelf's attachment points or area on the shell. Furthermore, the shelf acts as a separator within the shell between the container and the bottom tray. The shelf has an opening for bottles having a neck and shoulders, with the shoulders resting on the shelf, such that the neck with the connection to the feeding set can be located in the space above the bottom tray.

The height of the shelf can be adjusted such that sufficient space is available between the shelf and the bottom tray for the tubing of the feeding set not to form a kink. To this end, the side plates each comprise two or more ribs or slots, provided in pairs at two or more heights from the bottom tray, for positioning of the shelf. These heights are set to a space sufficient for most bottles attached to a feeding set without a drip chamber and to a set with a drip chamber. Further pairs of ribs or slots may be provided at intermittent heights, to account for smaller drip chambers or shorter bottle necks.

A further advantage of having the shelf attached to the side plates is that the shelf thereby provides additional stiffness to the carrier shell. Additionally, the shelf may comprise one or more locking elements at a back edge that can engage with the back plate of the shell. The back plate of the shell may comprise e.g. one or more locking element receiving slots located at two or more heights from the bottom tray for receiving the one or more locking elements. These locking element receiving slots may be positioned such that the locking element is locked inside a slot when the shelf is positioned on the ribs or in the slots. Locking the shelf within the shell increases its rigidity and ensures the carrier configuration remains as desired throughout mobile use.

The shelf may be slidably positionable over each set of ribs or into each set of slots by a movement in the direction of the ribs or slots, whereby the ribs or slots extend from the front edge of the side plates towards the back plate. The ribs or slots may be sloped downward towards the back plate to ensure the container will naturally rest against the back support. An angle of at least 5 degrees with respect to the bottom tray has proven to provide the desired effect. To increase ease of use, the shelf may be symmetric about its own plane such that the shelf can be used upside down without issues. Thus a user positioning the shelf within the shell need not pay attention to the orientation of the shelf in this respect and misplacement is avoided. This increases user convenience and reduces user errors. To further allow easy positioning of such a shelf, the shelf may be slightly triangular/trapezoidal in side view and the ribs may also be slightly triangular/trapezoidal to receive it. For an engagement that grips without jamming, an angle between the upper and lower surfaces of the shelf of between 5 degrees and 10 degrees may be chosen.

The adjustable container stabilizer enables the carrier to provide sufficient stabilizing support to containers which are sized or placed such that the centre of gravity of the container extends above the shell of the carrier. The adjustable container stabilizer makes the carrier suitable for use with containers of larger size otherwise not properly catered for by portable carrier frames, such as containers larger than 500 ml. Furthermore, this stabilizer allows for the carrier height to be increased only when required. This may be done independently from adjusting the height of the lower support element. Thus when small containers are being used, the height of the carrier can be kept at a minimum, providing ease of transport in a bag.

The stabilizer may be located within or against the back plate such that a longitudinal centreline of the stabilizer is parallel to the back plate, whereby the stabilizer is slidably movable along its centreline, and wherein the back plate further comprises container stabilizer locking elements, located at two or more heights from the bottom tray. The advantage of this configuration is that the stabilizer may be hidden fully within the back plate, thus allowing the carrier size to be limited to the size of the shell. By storing the stabilizer within the back plate, the stabilizer could add further stiffness to the shell. Furthermore, in this configuration the stabilizer can be a single extendable element, thus limiting the total number of components of the carrier and minimizing the overall weight of the carrier. The adjustable container stabilizer may be a plate with lockable elements.

Advantageously, a plate-like stabilizer located within the back plate is prevented from rotating around a longitudinal axis, adding further stability to the carrier and keeping the container in the set position.

Alternatively the adjustable container stabilizer may be a wireframe, formed out of a single continuous wire, of which the wire ends are lockable in the stabilizer locking elements. A wireframe could also be used for hanging products onto it, for example when bags are used as containers. An advantage of having a wireframe, formed out of a single wire is that such a wire has no sharp corners or sharp points, making the use of such a wireframe safe for the user and non-damaging to the bag in which the carrier may be transported.

Additionally, the container stabilizer may comprise a hanging hook, to facilitate hanging the carrier from a hook or infusion pole stand. It will be understood that in this case, the container stabilizer locking elements must prevent it from being further withdrawn from the back plate than desired.

To further add to the stability of the carrier, the container stabilizer locking elements may be spaced in pairs, symmetrically around the centreline and located at two or more heights from the bottom tray. The stabilizer locking elements may be simple slots or holes into which the lockable elements of the stabilizer may snap into place. This allows the stabilizer to be configured as an easy to use mechanical system, whereby the stabilizer can manually be pushed out of the locked positions for repositioning. These slots allow for easy, cheap and robust adjustability, wherein the stabilizer simply snaps into the locked positions. This is particularly convenient in the case that the stabilizer is a wire and the lockable elements are the wire ends. The resilience of the wire can be used to keep the lockable elements in the slots.

The back plate may be constructed with a top rib, which partially surrounds the stabilizer at the rear. The top rib may be positioned such that the stabilizer passes between a main surface of the back plate and the top rib. Such a top rib construction restricts the stabilizer to move primarily in a vertical direction, while simultaneously giving stiffness to the top edge of the shell to prevent bending e.g. when loaded with a container or subject to external forces.

According to a further embodiment, the back plate of the shell comprises a first set of strap slots and a second set of strap slots, the sets spaced in pairs around and along the longitudinal centreline of the shell, and wherein a distance between the centreline and the first set of strap slots is larger than a distance between the centreline and the second set of strap slots. The container retaining element may comprises at least one strap, connectable through a pair of strap slots, for encircling the container. Thus the strap slots may be set at different widths, allowing the container retaining element to more effectively hold various sized containers. The variable location of the container retaining element allows the carrier to accommodate various containers, including 125 ml bottles, 2 L pouches and baby-bottles.

Additionally, the container retaining element may further comprise at least one vertical strap, which is attached to the adjustable container stabilizer, and wherein the strap is adaptable to the size and shape of the container. An advantage of this configuration is that the strap moves automatically while adjusting the stabilizer.

In another embodiment the carrier further comprises an adjustable footrest, the foot rest being adjustable between a first position, wherein the footrest is within a perimeter of the shell, and a second position, wherein the footrest extends out from the shell, to a position where it can engage a surface on which the shell is placed, to stabilise the carrier. The adjustable footrest can be extended for use when the carrier is used as a stationary frame on for example a bed stand or table, outside a bag. As will be further described below, the shell is shaped to have a height greater than its base in order to adequately receive all of the elements that it is intended to support Advantageously, the extended footrest provides the carrier with a larger footprint, which adds to the stability of the shell. This is especially desirable when the carrier is used in combination with large containers, which cause the carrier to be top heavy and when the stabilizer is extended to a maximum.

The footrest may comprise a wire frame having dimensions corresponding to a horizontal cross-section of the shell above the bottom tray. Such wire frame is easy to store within the shell when not in use and due to its minimal dimensions, adds minimum weight to the carrier. Having a wire frame contributes to the carrier being lightweight, making it more comfortable for carrying around. For a carrier bottom tray with a square or rectangular shape, the wire frame may have a U-shape with diverging legs and a cross-piece. The legs may be stored within the shell and exit the back of the shell, with the cross-piece being stored on the outside of the shell, lying adjacent to or in a recess within the back surface of the back plate.

The shell may further comprise guides within which the footrest can slide. The guides define a path between the stored position and the extended, stabilising position. Extending the footrest is a simple manual operation whereby a user pulls on the accessible part of the footrest at the back of the carrier. The footrest then slides through the guides e.g. until the ends of the wire frame snap into the footrest locking points. These locking points may be slots or openings in the side plates of the carrier, located at the end of the guides. The footrest can be returned to the stored position by pushing both wire ends inward, freeing them from the locking points, and subsequent pushing the cross-piece of the wire frame toward the back plate of the shell, whereby the guides guide the footrest to the stored position.

The guides could be retention ribs or grooves, situated on the inner surfaces of the side plates of the shell. The guides can also extend towards the front of the shell to form an abutment surface for the front plate. Once the front plate is correctly affixed in position it may abut the abutment surfaces to prevent flexing. In an embodiment, the guides comprise webs, integrally formed with the side plates. These webs can have guide sections leading into the bearings. The guide section can guide the trunnions referenced above into the correct location within the bearings.

The carrier may further comprises one or more tubing guides for correctly guiding a tubing set to prevent kinking or entrapment. Tubing guides may be provided in one side plate and the back plate. Additionally or alternatively, a tubing guide may be provided on a rear side of the front plate. This is particularly desirable in the case that the pump is being used, allowing a user to insert the tubing into the tubing guide while the front plate is detached, thereby minimizing the tubing becoming kinked or entangled on reconnecting the front plate.

The shell of the carrier may be sized to fit inside a backpack and sized to hold a range of known enteral food and medical liquid containers. The shape of the shell is then optimized for comfortable carrying inside a backpack, whilst suitable for holding a range of containers. The carrier may be designed to fit a specific backpack or a backpack may be designed around the carrier. The carrier may also be carried in a bag of choice from the user, the bag being a backpack or any other type of bag fitting around the carrier. Whilst being carried in a bag, the shell provides rigidity to the bag and helps to protect the pump and container from being squashed. To aid comfortable carrying, the weight of the carrier is minimized such that the weight of the carrier is kept between 300 grams and 500 grams.

The shell may be dimensioned to have a height of between 20 cm and 50 cm, preferably between 23 cm and 26 cm. The width between the side plates may be between 10 cm and 30 cm, preferably between 13 cm and 15 cm. The bottom tray may have a depth from front to back of between 10 cm and 30 cm, preferably between 12cm and 15 cm. The side plates may extend forwards from the back plate by from 15 cm to 30 cm at the base and may taper upwards to between 5 cm and 10 cm at their upper limit. The bottom tray may have a depth i.e. measured in the height direction of the shell, between 0 cm and 3 cm, preferably between 0.5 cm and 2 cm, and any dish-shaped region is preferably sized to largely match the depth and width of the bottom plate. It may have a volume of from 50 cc to 200 cc, preferably as large as possible within the space available in order to maximise its ability to collect spillage. The height of the shelf compared to the bottom of the shell may be dimensioned between 7 - 25cm, depending on the type of reservoir and feeding set.

According to another aspect, the shell and front plate of the portable carrier may be made of dishwasher proof material, preferably an amorphous copolyester such as TritanTM. As a result, the whole carrier can be simply and hygienically cleaned in the dishwasher without discoloration or the shell material becoming damaged. The elements of the carrier could be made in an injection moulding process, allowing certain parts to be integrally formed. Furthermore, the shell could be made transparent, allowing visual control during use.

The present invention is further aimed at an assembly, comprising a carrier as described above or hereinafter, a container holding enteral food or a liquid, a feeding set and a feed pump. The feeding set may be with or without a drip chamber. The assembly may also comprise a bag in which elements of the assembly are contained, wherein the bag has a base adapted to fit the bottom tray or wherein the bag is sized to snuggly receive the shell. The bag is preferably a backpack.

### Brief description of the drawings

The invention will be discussed in more detail below, with reference to the attached drawings in which illustrative embodiments thereof are shown. The drawings are intended exclusively for illustrative purposes and not as a restriction of the inventive concept. The scope of the invention is only limited by the definitions presented in the appended claims.
Figure 1 depicts a mobile enteral feed assembly including a carrier according to the invention.
Figure 2A shows an isometric view of the front side of the carrier of Figure 1.
Figure 2B shows the view of Figure 2A including the front plate.
Figure 2C shows the front plate of Figure 2B from the rear.
Figure 3 shows an isometric view of the rear side of the carrier of Figure 2A, with extended container stabilizer and footrest.
Figure 4A shows an isometric frontal view of a container rest shelf for a carrier according to the invention.
Figure 4B shows an isometric rear view of a container rest shelf for a carrier according to the invention.
Figure 5 shows an assembly of the carrier with the feed pump.
Figure 6 shows the feed pump with the front plate being used as a table stand.

### Description of embodiments

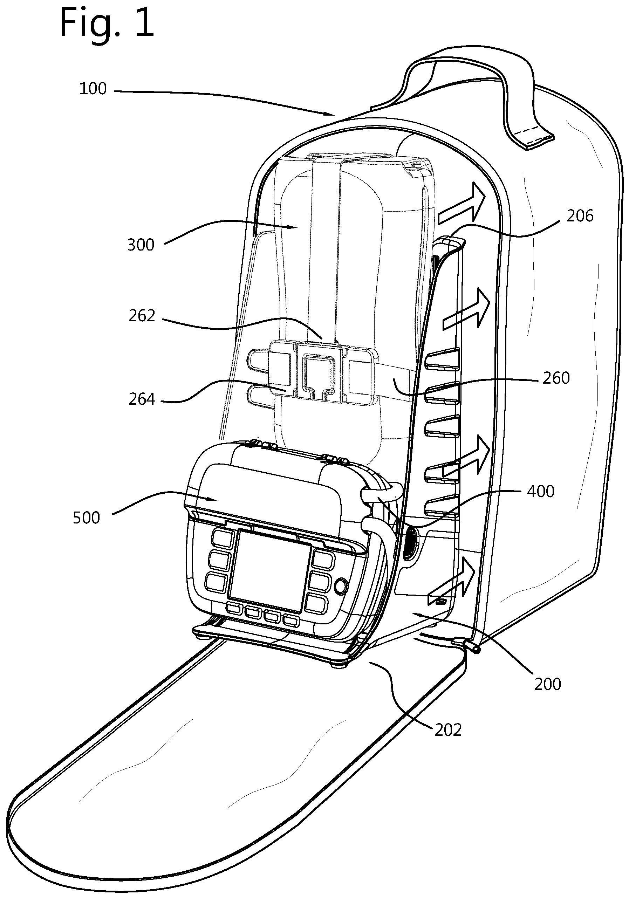

Figure 1 depicts a mobile enteral feed assembly 1 according to the invention. The assembly 1 comprises a backpack 100, a carrier 200, an enteral feed or liquid container 300, feeding set 400 and a feed pump 500.

The enteral feed or liquid container 300 is connected to the feeding set 400 at its main opening and placed upside-down into the carrier 200. Due to the upside-down placement of the container 300, the enteral feed or liquid will naturally flow into the tubing 400 under gravity. The carrier 200 is designed for offering good stability to the container and equipped with fastening means which hold the container in a predetermined position within the carrier.

The feeding set 400 is run through the feed pump 500, wherein the pump is set up to pump the enteral food or liquid from the container through the feeding set 400 at a predetermined rate towards a patient carrying the assembly. The feed pump 500 may be any conventional feed pump such as the Flocare Infinity TM pump available from Nutricia and is not further part of the present invention.

The carrier 200 forms a protective shell, partially surrounding the container and the feed pump. Furthermore, the carrier 200 is shaped to fit into a backpack 100, such that the back plate of the carrier ergonomically sits against the back of a person carrying the backpack 100 and the bottom tray of the carrier keeps the carrier stable within the bag, while preferably also serving as a spillage tray, keeping the bag clean.

The backpack 100 shown in this example is a backpack specifically designed to hold the carrier 200 with the container and feed pump. This specifically designed backpack illustrated includes a large access zip for accessing, inserting and removing the entire carrier and a smaller access zip for accessing the control panel of the feed pump. Alternatively, the backpack could be any bag chosen by the patient.

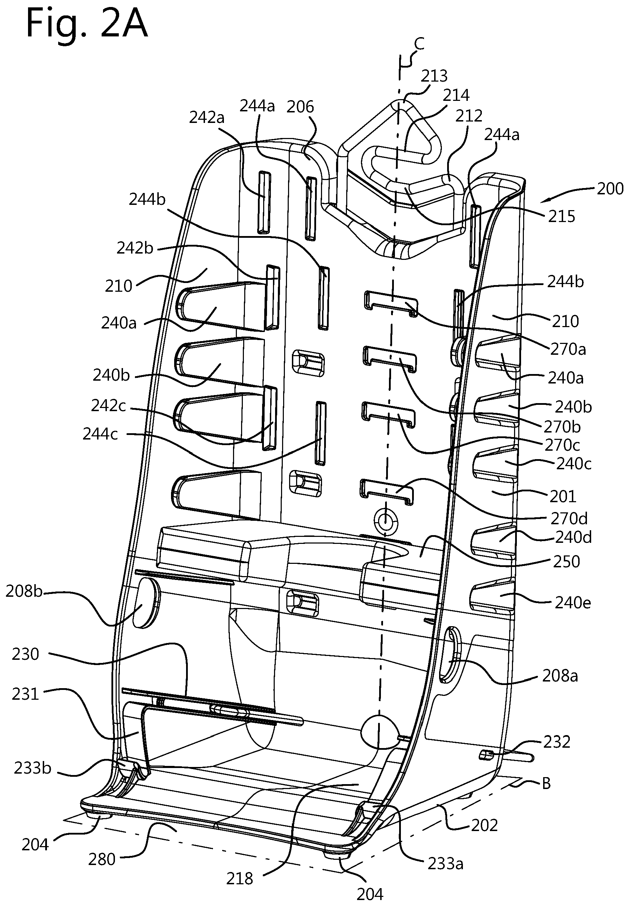

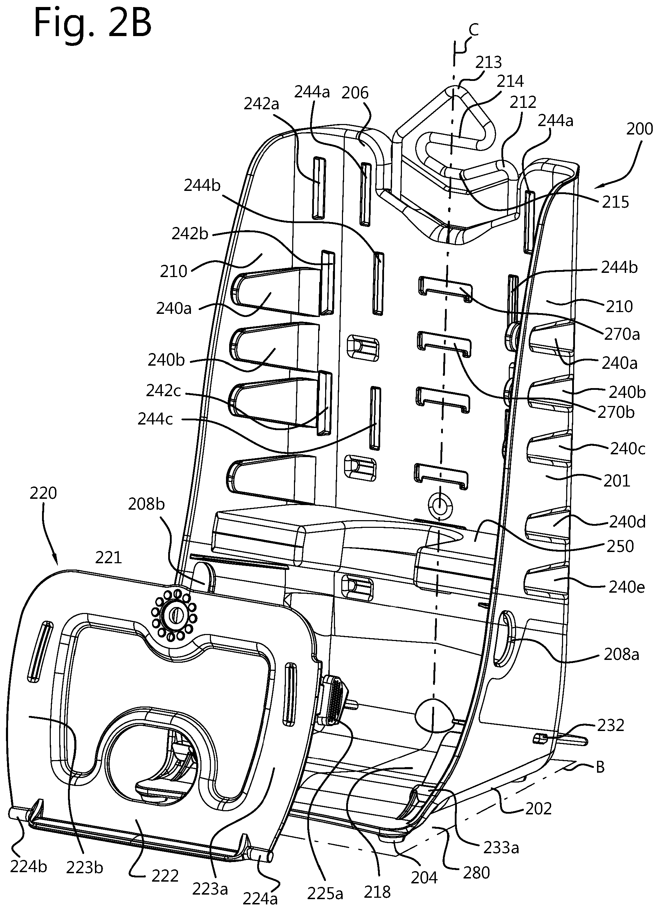

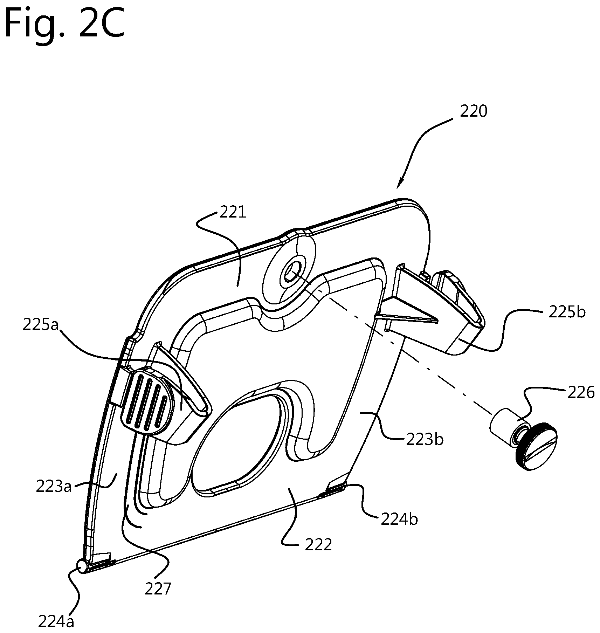

More details of the carrier 200 are explained in reference to Figures 2 - 4.

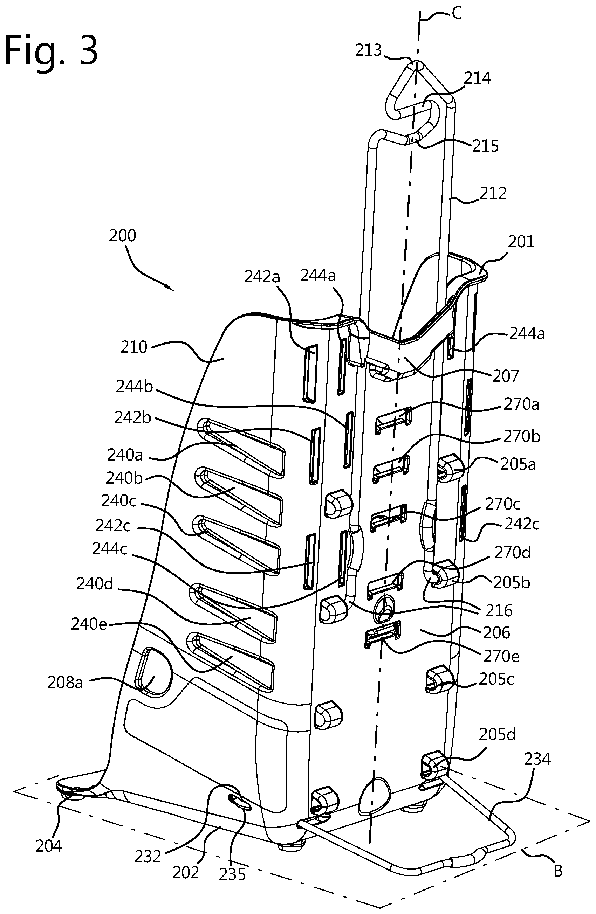

Figures 2A, 2B and 3 show isometric views of the front and back of a carrier 200 according to the invention. The carrier 200 comprises a shell 201 with a front plate 220 and a repositionable container shelf 250, whereby the shell 201 is further equipped with an adjustable container stabilizer 212 and an adjustable footrest 234. The shell 201 has a bottom tray 202, a back plate 206 and two side plates 210.

The shell's bottom tray 202 is shaped to form a stable base to the carrier 200, both when carried in a backpack 100 and when used as a table top stand. To this end, the front and rear corner areas of the bottom tray are shaped to rest within the same plane B, whereby at least the front bottom tray corners contain footrests 204. Furthermore, the bottom tray 202 includes a spillage tray 218 for collecting any enteral food or liquid which may be spilled from the connection between the container 300 and the feeding set 400, thus keeping the backpack 100 or table on which the carrier is used clean.

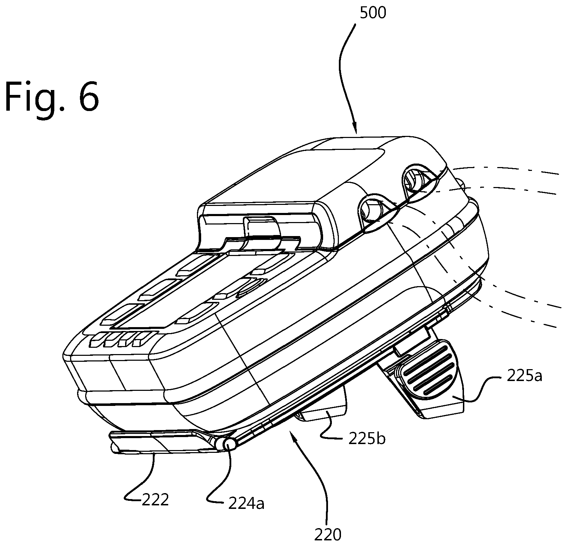

The shell's back plate 206 extends substantially perpendicular to the bottom tray 202 from the back edge of the bottom tray 202. The back plate 206 comprises wide strap openings 242 and narrow strap openings 244, shelf locking openings 270 and stabilizer locking elements 205. The strap openings 242, 244 form vertical slots with respect to the bottom tray 202, which openings are provided for slotting through container holding straps (see Figure 6). The wide strap openings 242 are set at a wider distance around a centreline C of the back plate 206 than the narrow strap openings 244, allowing for tight strapping of both wide and narrow containers. This makes the container carrier also suitable for use with, for example, baby-bottles. The wide strap openings 242 include three pairs of strap openings 242 a-c set at three different heights with respect to the bottom tray 202, which allow the strap to be placed according to the height of the container. The same applies to the narrow strap openings 244 a-c. It will be understood that alternative numbers and distributions of slots or other alternative attachment provisions may be provided.

The shelf locking openings 270 a-e in this example are provided as a row of horizontal slots with respect to the bottom tray 202, placed symmetrically on the centreline C of the back plate. The shelf locking openings 270 a-e are set at five different heights with respect to the bottom tray 202. The height at which each opening 70 a, b is placed corresponds to a predetermined height at which a container 300 can be placed into the carrier 200. The stabilizer locking elements 205 are described in more detail in relation to the adjustable stabilizer element 212.

The side plates 210 of the shell 201 extend substantially perpendicular to the bottom tray 202 and to the back plate 206. The side plates 210 are provided with left and right apertures 208 a,b for receiving the front plate 220 as described further below. The side plates 210 have a width which is large enough to cover the depth of most used enteral food and other liquid containing containers. The shell 201 is triangular/trapezoidal in side view. As a result, the shell 201 provides substantial protection to the containers whilst being carried in a bag, while still allowing good access to the container.

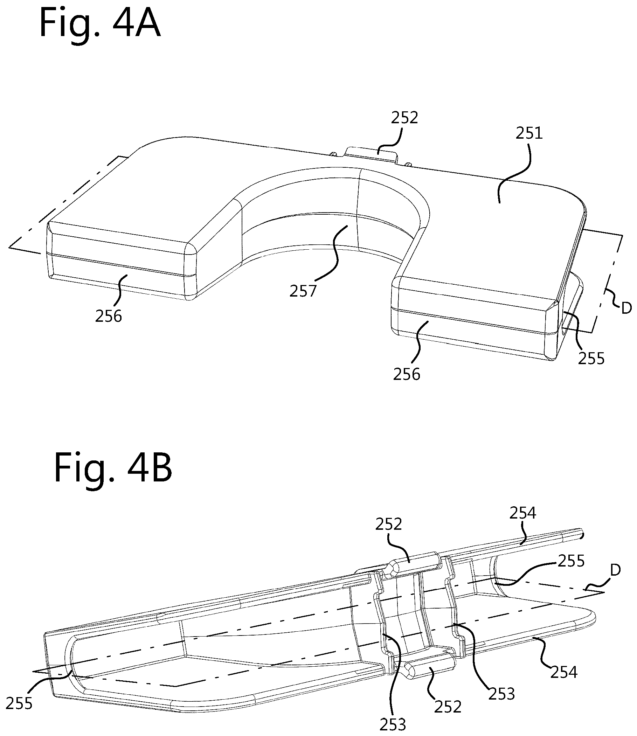

Further, the side plates 210 each comprise positioning ribs 240, a guide web 230 and a locking opening 232. The guide webs 230 extend forwards to form guide sections 231, which extend downwards to bearings 233a,b. The guide web 230 and locking opening 232 are described in more detail in relation to the adjustable footrest 234 below. The positioning ribs 240 a-e are placed in pairs divided over the two side plates 210, such that the side plates 210 mirror each other with respect to centreline C, for supporting a container rest shelf 250 (described in more detail in the figure description for Figures 4A and 4B). The positioning ribs 240 a-e are shaped to match with the shape of the container rest shelf 250, whereby the shelf 250 contributes positively to the stiffness of the shell 201. Additionally, the positioning ribs 240 a-e are placed such that the container rest shelf 250 is fitted at an angle of at least 5 degrees compared to the bottom tray 202, allowing the container to rest naturally against the back plate 206. Furthermore, the positioning ribs 240 a-e are located at five different heights with respect to the bottom tray, allowing the shelf 250 to be placed at five different heights. These heights are predetermined such that the carrier 200 remains as compact as possible, whilst providing sufficient space between the shelf 250 and the bottom tray 202 such that no kinks occur in the feeding set 400. A first pair of ribs 240e is placed at the lowest height, for use of a container with a feeding set without a drip chamber. This hight corresponds to the upper edge of the front plate 220. The aforementioned openings 270 a-e in the back plate 206 are matched to the height of the pairs of positioning ribs 240 a-e.

The front plate 220 is shown in Figure 2C from the rear. It comprises an upper edge 221, a lower edge 222 and two side edges 223a,b. The front plate 220 also has left and right trunnions 224a,b and clips formed by left and right fingers 225a,b. The front plate 220 attaches releasably to the shell 201, whereby the fingers 225a,b engage within the apertures 208a,b and the trunnions 224a,b are received within the bearings 233a,b. The front plate 220 is also provided with a screw connector 226 visible at its rear surface, which is sized and located to correspond with a threaded socket (not shown) on a rear surface of the feed pump. Also visible on the rear surface of the front plate 220 is a tubing guide 227. The tubing guide 227 retains the tubing when the front plate is being installed and reduces the risk of the tubing kinking or being trapped.

The shell 201 is formed as a single piece, manufactured in one injection moulding step of a rigid and transparent, amorphous copolyester material available as TritanTM from Eastman corp. It is dishwasher proof and can thus be easily washed after removal from the bag 100. It will be understood that other plastic materials may be used, including both rigid and semi rigid plastics.

As previously listed, the portable carrier 200 depicted in Figures 2 and 3 further comprises an adjustable stabilizer element 212. The stabilizer element 212 is a longitudinal element, comprising a hanging hook 213, a horizontal section 214 and a holding strap connection bar 215 on one end which extends out from the top edge of the back plate 206, and connector elements 216 on the other end. In the present example, the stabilizer element 212 is a wire frame, shaped from a single piece of wire, whereby the wire ends form the connector elements 216. The stabilizer element 212 is located at or (partially) in the back plate 206 of the shell 201, such that a longitudinal centreline of the stabilizer substantially coincides with a longitudinal centreline C of the back plate 206. The stabilizer element 212 is installed in/onto the back plate 206 such that the stabilizer element 212 is movable only along the centreline C, between a first position wherein the stabilizer element 212 is mostly hidden in/behind the back plate 206 with only the hook 213, horizontal section 214 and holding strap connection bar 215 extending out above the top edge of the back plate 206, and a second position in which the stabilizer element 212 mostly extends above the top edge of the back plate 206, such that only the bottom end comprising the connector elements 216 remains in/behind the back plate 206. To ensure the alignment of the stabilizer element 212 and the back plate 206, and to further prevent any rotation of the stabilizer element 212 with respect to the back plate 206, the upper part of the back plate comprises a stabilizer rib 207. This stabilizer rib 207 is integrally formed with the shell 201 and lies in a plane parallel to the back plate 206, such that the back plate 206 is at a first side of the stabilizer element 212 and the stabilizer rib 207 is at a second, opposing side of the stabilizer element 212. The stabilizer element 212 is thus partially surrounded and enclosed by the stabilizer rib 207. Further, the stabilizer element 212 can be locked in position at at least two different heights, using the previously mentioned stabilizer locking elements 205 a-d, located in the back plate 206. In the present example, the back plate 206 is supplied with four pairs of locking elements 205 a-d, placed symmetrically around the centreline C of the plate. The locking elements 205 a- d are set at four different heights with respect to the bottom tray 202, allowing the stabilizer element 212 to be locked at four different extension levels with respect to the back plate 206.

The higher locking positions 205 a, b effectively increase the supported height provided by the carrier 200, and can be used to stabilize large containers, which may be larger than 500 ml. Furthermore, the stabilizer element 212 provides additional utility in that hook 213 may be used further as carrier hand grip or for hanging containers from. Finally, due to the smooth and continuous shape of the wire, the stabilizer element 212 does not have any sharp and/or protruding ends, the stabilizer element is non-damaging to the carrier bag and safe for the user to walk around with.

Lastly, the portable carrier 200 comprises the previously referenced footrest 234. In the present example, the footrest 234 is a bracket shaped wire, which is shaped to match a cross-sectional shape of the shell 201. The footrest 234 is spaced from the bottom plane B and set at a downward angle towards the back plate 206. The ends of the bracket shaped wire are shaped to form locking elements 235.

The footrest 234 is positioned inside a bottom section of the shell 201, below the lowest position of the container support shelf 250, such that the footrest 234 is slidably deployable from the back of the shell 201. In the stored position, the footrest 234 is located entirely within the perimeter of the shell 201, whereby the two extending legs of the bracket shaped wire 234 are positioned within the shell 201, located in footrest guide webs 230 which are integral with the side plates 210 and angled downward towards the back plate 206. When deploying the footrest 234 to its extended position, the legs of the bracket slide through the footrest guide webs 230, until reaching a final locked position, wherein the locking elements 235 snap into locking holes 232. Each side plate 210 comprises a locking hole 232 at the end of the footrest guide web 230 near the back plate 206. In this extended and locked position, the position of the footrest 234 with respect to the shell 201 is such that the back plate matching section of the bracket shaped wire rests in bottom plane B. The footrest 234 can be returned to the retracted position by pushing the locking elements 235 back into the locking holes 232 and sliding the bracket back inwards.

By extending the footrest 234, the footprint of the carrier 200 is increased, which further improves the stability of the carrier 200. This further improved stability allows the carrier 200 to be used safely as a table stand with heavier and larger containers filled with enteral food or liquid, which otherwise would cause the stand to become unstable due to having a higher centre of gravity.

Figures 4A and 4B show isometric front and rear views of the container rest shelf 250. The container rest shelf 250 has a front edge 256 with a recess 257, rear edges 254, side edges 255, a container rest surface 251 and a locking element comprising broad lip locks 252 and stability ribs 253. The container rest shelf 250 is symmetric with respect to a plane D, which extends from and is perpendicular to the front edge 256 between the broad lip locks 252. As a result, the shelf fits into the carrier, regardless if the presently visible resting surface 251 is facing up or down. Furthermore, the side edges 255 are shaped to fit around the table stand ribs 240 such that the ribs add stiffness to the rest shelf 250.

When fully installed over a pair of positioning ribs 240, the upper broad lip lock 252 is locked into a matching shelf locking opening 270. The stability ribs 253 add stiffness and stability to the back of the shelf 250, such that the broad lip lock 252 is prevented from bending under pressure from a container resting on the top surface 251. Due to the angle of at least 5 degrees at which the positioning ribs 240 are placed, the bottom lip lock 252 sits adjacent to the back plate 206, when the shelf 250 is locked in position in the shell 201. The container rest shelf 250 can be made from the same material and using the same manufacturing method as the shell 201. The shelf 250 is also preferably made of a dishwasher proof material.

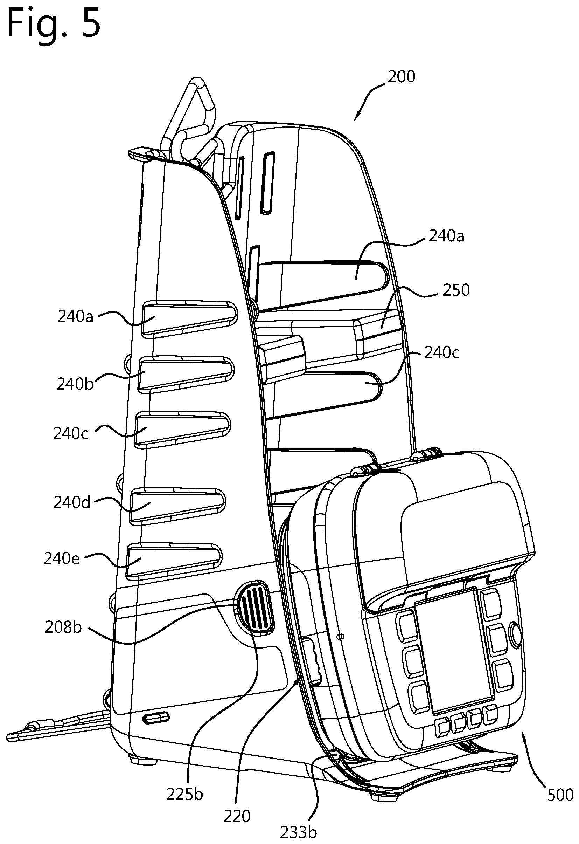

Figure 5 shows an assembly of a carrier 200 according to the invention and a feed pump 500. The feed pump 500 is affixed to the front plate 220 with the fingers 225 engaged in the apertures 208. The container support shelf 250 is located at the highest table stand ribs 240a.

Figure 6 shows the feed pump 500 with the front plate 220 separated from the shell 201 being used as a table stand. The front plate 220 rests on its lower edge 222 and left and right fingers 225a,b. In this position, the feed pump is oriented at around 20 degrees to the horizontal, which significantly improves readability, especially when viewed from a seated position or in bed.

Further obvious modifications and alterations will occur to others upon reading and understanding the preceding detailed description. It is intended that the invention be construed as including all such modifications and alterations insofar as they come within the scope of the appended claims.

## Claims

1. A carrier (200) for transporting and administering enteral food or other liquids provided in a container, the carrier comprising
a shell (201), comprising a bottom tray (202), a back plate (206) and two side plates (210) and a feed pump holding element;
support elements for holding the container in place within the shell, the support elements comprising an adjustable container stabilizer (212), extendable vertically with respect to the back plate
**characterised in that** the feed pump holding element comprises a removable front plate (220), releasably secured between the side plates and having a front face onto which a feed pump can be attached, the front plate comprising an upper edge (221), a lower edge (222) and two side edges (223), the side edges being provided with clips located adjacent to the upper edge for engaging the side plates, the clips extending rearwardly from the front plate as feet on which the front plate can stand at an angle once removed from the shell and the front plate is further attached to the shell adjacent to its lower edge in a pivotable connection.

2. The carrier according to claim 1, wherein the side plates are provided with left and right apertures (208) and the clips comprise resilient fingers (225) arranged to extend into the apertures from within the shell.

3. The carrier according to claim 2, wherein the resilient fingers are accessible from an outside of the shell to release them from the apertures in order to disengage the front plate.

4. The carrier according to any one of the preceding claims, wherein the front plate comprises left and right trunnions (224) adjacent its lower edge that engage in bearings (233) formed in the side plates.

5. The carrier according to any one of the preceding claims, wherein a rear surface of the front plate comprises a tubing guide (227) for retaining a portion of enteral feed tubing.

6. The carrier according to any one of the preceding claims, wherein a screw connector is (226) provided on a rear surface of the front plate for releasably attaching the feed pump.

7. The carrier according to any one of the previous claims, wherein the bottom tray comprises a spillage tray (218) and a lower edge of the front plate extends forwards of the spillage tray.

8. The carrier according to any one of the previous claims, wherein the adjustable container stabilizer is extendable to a position at which a centre of gravity of the container extends above the shell of the carrier.

9. The carrier according to any preceding claim, wherein the container stabilizer comprises a hanging hook (213).

10. The carrier according to any one of the previous claims, further comprising an adjustable footrest (234), the footrest being adjustable between a first position, wherein the footrest is within a perimeter of the shell, and a second position, wherein the footrest extends out from the shell to a position where it can engage a surface on which the shell is placed to stabilise the carrier, the side plates comprising guides within which the footrest can slide, the guides forming an abutment surface for the front plate.

11. The carrier according to claim 4 and claim 10, wherein the guides comprise webs (230), integrally formed with the side plates and having guide sections leading into the bearings.

12. The carrier according to any preceding claim, wherein the support elements comprise a lower support element for location beneath the container and a container retaining element for retaining the container in position within the shell, wherein the lower support element is height adjustable with respect to the bottom tray, thereby allowing a container to be supported at different positions above the bottom tray, optionally wherein the lower support element comprises a container rest shelf (250), extending between the side plates and a lowest position of the shelf is aligned with an upper edge of the front plate.

13. A front plate (220) for use in a carrier according to any preceding claim, the front plate being arranged to securely connect to the rear face of a feed pump and comprising an upper edge (221), a lower edge (222) and two side edges 223), the side edges having a pair of clips for attachment to a carrier shell, **characterised in that** the clips are located adjacent to the upper edge and extend rearwardly from the front plate as feet (225), a distance to allow the front plate to stand on the clips with the feed pump at an angle of between 15 degrees and 45 degrees on a horizontal surface once removed from the shell and the front plate further comprises a hinge adjacent to its lower edge for pivotable connection to the shell.

14. An assembly, comprising:
the carrier (200) according to any one of claims 1 to 12;
a container (300) holding enteral food or a liquid;
a feeding set (400); and
a feed pump (500) attached to a front surface of the front plate.

15. Assembly according to claim 14, further comprising a backpack (100), having internal dimensions corresponding to outside dimensions of the shell, optionally wherein the backpack has an openable front panel, giving access to the feed pump.

## Patentansprüche

1. Träger (200) zum Transportieren und Verabreichen von enteraler Nahrung oder anderen Flüssigkeiten, die in einem Behälter bereitgestellt werden, wobei der Träger Folgendes umfasst
ein Gehäuse (201), umfassend eine Bodenwanne (202), eine Rückplatte (206) und zwei Seitenplatten (210) sowie ein Halteelement für eine Zufuhrpumpe;
Stützelemente zum Halten des Behälters an seinem Platz innerhalb des Gehäuses, wobei die Stützelemente einen verstellbaren Behälterstabilisator (212) umfassen, der vertikal in Bezug auf die Rückplatte verlängerbar ist,
**dadurch gekennzeichnet, dass** das Halteelement für die Zufuhrpumpe eine abnehmbare Frontplatte (220) umfasst, die lösbar zwischen den Seitenplatten befestigt ist und eine Vorderseite aufweist, an der eine Zufuhrpumpe befestigt werden kann, wobei die Frontplatte eine Oberkante (221), eine Unterkante (222) und zwei Seitenkanten (223) umfasst, wobei die Seitenkanten mit Klammern versehen sind, die benachbart zu der Oberkante zum Eingriff mit den Seitenplatten angeordnet sind, wobei sich die Klammern als Füße von der Frontplatte nach hinten erstrecken, auf denen die Frontplatte in einem Winkel stehen kann, sobald sie aus dem Gehäuse entfernt wurde, und wobei die Frontplatte zusätzlich benachbart zu ihrer Unterkante in einer schwenkbaren Verbindung an dem Gehäuse befestigt ist.

2. Träger nach Anspruch 1, wobei die Seitenplatten mit linken und rechten Öffnungen (208) versehen sind und die Klammern federnde Finger (225) umfassen, die dazu angeordnet sind, sich von innerhalb des Gehäuses in die Öffnungen zu erstrecken.

3. Träger nach Anspruch 2, wobei die federnden Finger von einer Außenseite des Gehäuses zugänglich sind, um sie aus den Öffnungen zu lösen, um die Frontplatte außer Eingriff zu bringen.

4. Träger nach einem der voranstehenden Ansprüche, wobei die Frontplatte linke und rechte Lagerzapfen (224) benachbart zu ihrer Unterkante umfasst, die in Lager (233) eingreifen, die in den Seitenplatten ausgebildet sind.

5. Träger nach einem der voranstehenden Ansprüche, wobei eine Rückseite der Frontplatte eine Schlauchführung (227) zum Zurückhalten eines Abschnitts eines enteralen Zufuhrschlauchs umfasst.

6. Träger nach einem der voranstehenden Ansprüche, wobei ein Schraubverbinder (226) an einer Rückseite der Frontplatte zum lösbaren Befestigen der Zufuhrpumpe vorgesehen ist.

7. Träger nach einem der vorherigen Ansprüche, wobei die Bodenwanne eine Auffangwanne (218) umfasst und eine Unterkante der Frontplatte sich nach vorne von der Auffangwanne erstreckt.

8. Träger nach einem der vorherigen Ansprüche, wobei der verstellbare Behälterstabilisator in eine Position verlängerbar ist, in der sich ein Schwerpunkt des Behälters über das Gehäuse des Trägers erstreckt.

9. Träger nach einem der voranstehenden Ansprüche, wobei der Behälterstabilisator einen Aufhängehaken (213) umfasst.

10. Träger nach einem der vorherigen Ansprüche, weiter umfassend einen verstellbaren Stützfuß (234), wobei der Stützfuß zwischen einer ersten Position, in der sich der Stützfuß innerhalb eines Umfangs des Gehäuses befindet, und einer zweiten Position verstellbar ist, in der sich der Stützfuß aus dem Gehäuse heraus in eine Position erstreckt, in der er mit einer Oberfläche, auf der das Gehäuse platziert ist, in Eingriff treten kann, um den Träger zu stabilisieren, wobei die Seitenplatten Führungen umfassen, innerhalb derer der Stützfuß gleiten kann, wobei die Führungen eine Anschlagfläche für die Frontplatte bilden.

11. Träger nach Anspruch 4 und Anspruch 10, wobei die Führungen Stege (230) umfassen, die einstückig mit den Seitenplatten ausgebildet sind und Führungsabschnitte aufweisen, die in die Lager führen.

12. Träger nach einem der voranstehenden Ansprüche, wobei die Stützelemente ein unteres Stützelement zur Anordnung unterhalb des Behälters und ein Behälterrückhalteelement zum Halten des Behälters in Position innerhalb des Gehäuses umfassen, wobei das untere Stützelement in Bezug auf die Bodenwanne höhenverstellbar ist, wodurch ein Behälter an verschiedenen Positionen über der Bodenwanne abgestützt werden kann, wobei optional das untere Stützelement ein Behälterauflagefach (250) umfasst, das sich zwischen den Seitenplatten erstreckt, und eine unterste Position des Fachs mit einer Oberkante der Frontplatte ausgerichtet ist.

13. Frontplatte (220) zur Verwendung in einem Träger nach einem der voranstehenden Ansprüche, wobei die Frontplatte dazu angeordnet ist, sicher mit der Rückseite einer Zufuhrpumpe verbunden zu werden, und eine Oberkante (221), eine Unterkante (222) und zwei Seitenkanten (223) umfasst, wobei die Seitenkanten ein Paar Klammern zur Befestigung an einem Trägergehäuse aufweisen, **dadurch gekennzeichnet, dass** die Klammern benachbart zu der Oberkante angeordnet sind und sich als Füße (225) von der Frontplatte nach hinten erstrecken, und zwar über eine Distanz, die es der Frontplatte ermöglicht, auf den Klammern mit der Zufuhrpumpe in einem Winkel zwischen 15 Grad und 45 Grad auf einer horizontalen Fläche zu stehen, sobald sie aus dem Gehäuse entfernt wurde, und wobei die Frontplatte weiter ein Scharnier benachbart zu ihrer Unterkante zur schwenkbaren Verbindung mit dem Gehäuse umfasst.

14. Anordnung, umfassend:
den Träger (200) nach einem der Ansprüche 1 bis 12;
einen Behälter (300), der enterale Nahrung oder eine Flüssigkeit enthält;
ein Zufuhrset (400); und
eine Zufuhrpumpe (500), die an einer Vorderseite der Frontplatte befestigt ist.

15. Anordnung nach Anspruch 14, weiter umfassend einen Rucksack (100), der Innenabmessungen aufweist, die Außenabmessungen des Gehäuses entsprechen, wobei der Rucksack optional eine Vorderwand aufweist, die geöffnet werden kann und Zugang zu der Zufuhrpumpe gewährt.

## Revendications

1. Support (200) pour transporter et administrer des aliments entéraux ou d'autres liquides disposés dans un récipient, le support comprenant
une coque (201), comprenant un plateau inférieur (202), une plaque arrière (206) et deux plaques latérales (210) et un élément de maintien de la pompe d'alimentation ;
des éléments de support pour maintenir le récipient en place dans la coque, les éléments de support comprenant un stabilisateur de récipient réglable (212), extensible verticalement par rapport à la plaque arrière
**caractérisé en ce que** l'élément de maintien de la pompe d'alimentation comprend une plaque avant amovible (220), fixée de manière libérable entre les plaques latérales et présentant une face avant sur laquelle une pompe d'alimentation peut être attachée, la plaque avant comprenant un bord supérieur (221), un bord inférieur (222) et deux bords latéraux (223), les bords latéraux étant pourvus d'attaches situées de manière adjacente au bord supérieur pour s'engager avec les plaques latérales, les attaches s'étendant vers l'arrière depuis la plaque avant sous la forme de pieds sur lesquels la plaque avant peut reposer de manière inclinée une fois retirée de la coque et la plaque avant est en outre attachée à la coque de manière adjacente à son bord inférieur par une liaison pivotante.

2. Support selon la revendication 1, où les plaques latérales sont pourvues d'ouvertures gauches et droites (208) et les attaches comprennent des pattes élastiques (225) agencées de façon à s'étendre dans les ouvertures depuis l'intérieur de la coque.

3. Support selon la revendication 2, où les pattes élastiques sont accessibles depuis un extérieur de la coque pour les libérer des ouvertures afin de désengager la plaque avant.

4. Support selon l'une quelconque des revendications précédentes, où la plaque avant comprend des tourillons gauche et droit (224) adjacents à son bord inférieur qui s'engagent dans des paliers (233) formés dans les plaques latérales.

5. Support selon l'une quelconque des revendications précédentes, où une surface arrière de la plaque avant comprend un guide de tubulure (227) pour la retenue d'une partie de la tubulure d'alimentation entérale.

6. Support selon l'une quelconque des revendications précédentes, où un connecteur à vis (226) est pourvu sur une surface arrière de la plaque avant pour attacher de manière libérable la pompe d'alimentation.

7. Support selon l'une quelconque des revendications précédentes, où le plateau inférieur comprend un bac de récupération des déversements (218) et un bord inférieur de la plaque avant s'étend en avant du bac de récupération des déversements.

8. Support selon l'une quelconque des revendications précédentes, où le stabilisateur de récipient réglable est extensible jusqu'à une position à laquelle un centre de gravité du récipient dépasse la coque du support.

9. Support selon l'une quelconque des revendications précédentes, où le stabilisateur de récipient comprend un crochet de suspension (213).

10. Support selon l'une quelconque des revendications précédentes, comprenant en outre un repose-pieds réglable (234), le repose-pieds étant réglable entre une première position, où le repose-pieds se trouve à l'intérieur d'un périmètre de la coque, et une deuxième position, où le repose-pieds s'étend hors de la coque jusqu'à une position où il peut s'engager sur une surface sur laquelle la coque est placée pour stabiliser le support, les plaques latérales comprenant des guides dans lesquels le repose-pieds peut glisser, les guides formant une surface de butée pour la plaque avant.

11. Support selon la revendication 4 et la revendication 10, où les guides comprennent des nervures (230), formées d'un seul bloc avec les plaques latérales et présentant des sections de guidage débouchant dans les paliers.

12. Support selon l'une quelconque des revendications précédentes, où les éléments de support comprennent un élément de support inférieur destiné à être placé sous le récipient et un élément de retenue de récipient destiné à retenir le récipient en position dans la coque, où l'élément de support inférieur est réglable en hauteur par rapport au plateau inférieur, permettant ainsi de supporter un récipient à différentes positions au-dessus du plateau inférieur, facultativement où l'élément de support inférieur comprend une étagère de repos (250) pour récipient, s'étendant entre les plaques latérales et une position la plus basse de l'étagère est alignée avec un bord supérieur de la plaque avant.

13. Plaque avant (220) pour une utilisation dans un support selon l'une quelconque des revendications précédentes, la plaque avant étant agencée pour se relier de manière fixe à la face arrière d'une pompe d'alimentation et comprenant un bord supérieur (221), un bord inférieur (222) et deux bords latéraux (223), les bords latéraux ayant une paire d'attaches pour s'attacher à une coque de support, **caractérisée en ce que** les attaches sont situées de manière adjacente au bord supérieur et s'étendent vers l'arrière depuis la plaque avant sous la forme de pieds (225), une distance permettant à la plaque avant de reposer sur les attaches avec la pompe d'alimentation inclinée entre 15 degrés et 45 degrés sur une surface horizontale une fois retirée de la coque, et la plaque avant comprend en outre une charnière adjacente à son bord inférieur pour une liaison pivotante avec la coque.

14. Ensemble, comprenant :
le support (200) selon l'une quelconque des revendications 1 à 12 ;
un récipient (300) contenant des aliments entéraux ou un liquide ;
un ensemble d'alimentation (400) ; et
une pompe d'alimentation (500) attachée à une surface avant de la plaque avant.

15. Ensemble selon la revendication 14, comprenant en outre un sac à dos (100), ayant des dimensions internes correspondant aux dimensions externes de la coque, facultativement où le sac à dos a un panneau avant ouvrable, donnant accès à la pompe d'alimentation.
